# DEMANDE DE BREVET EUROPEEN

(11) **EP 1 110 539 A1**
(43) Date de publication de la demande: **27.06.2001**
(21) Numéro de dépôt: 00403528.3
(22) Date de dépôt: 14.12.2000
(51) Int. Cl.: A61K 7/48

(54) **Utilisation du 3,3', 5,5'-tétrahydroxystilbène comme agent anti-glycation**

(30) Priorité: 21.12.1999 FR 9916168
(71) Demandeur: L'OREAL, 75008 Paris (FR)
(72) Inventeur: Maignan, Jean, 93290 Tremblay-en-France (FR)
(74) Mandataire: Galup, Cédric Olivier Nicolas

(57) **Abrégé**

L'invention se rapporte à l'utilisation du 3,3',5,5'-tétrahydroxystilbène ou de l'un de ses dérivés dans ou pour la préparation d'une composition, l'hydroxystilbène ou la composition étant destinés à diminuer voire inhiber la glycation des protéines, particulièrement des protéines de la peau et/ou de ses annexes.

## Description

L'invention se rapporte à l'utilisation du 3,3',5,5'-tétrahydroxystilbène ou de l'un de ses dérivés dans ou pour la préparation d'une composition, l'hydroxystilbène ou la composition étant destinés à diminuer voire inhiber la glycation des protéines, particulièrement des protéines de la peau et/ou de ses annexes.

La glycation est un processus non enzymatique faisant intervenir un ose (glucose ou ribose) qui réagit selon la réaction de Maillard avec un groupement aminé d'un résidu d'acide aminé (comme par exemple la lysine), particulièrement un résidu d'acide aminé d'une protéine, pour former une base de Schiff. Celle-ci, après un réarrangement moléculaire dit d'Amadori, peut conduire, par une succession de réactions, à un pontage, particulièrement intramoléculaire comme par exemple de type pentosidine.
Ce phénomène augmente de façon régulière avec l'âge. Il se caractérise par l'apparition de produits de glycation dont la teneur augmente de façon régulière en fonction de l'age. Les produits de glycation sont par exemple la pyrraline, la carboxyméthyl-lysine, la pentosidine, la crossline, la N^{ε}(2carboxyéthyl)-lysine (CEL), la glyoxal-lysine dimer (GOLD), la méthylglyoxallysine dimer (MOLD), la 3DG-ARG imidazolone, les versperlysines A, B, C, la thréosidine ou encore les produits terminaux de glycosylation avancée (advanced glycosylation ends products ou AGEs).
La glycation des protéines est donc un phénomène universel, bien connu au niveau de la peau, particulièrement au niveau de sa composante dermique, mais qui survient également dans les annexes de celle-ci comme les ongles ou les cheveux, particulièrement sur les kératines et plus généralement dans tout système protéique pour peu que les conditions requises pour la glycation soient réunies.

La peau humaine est constituée de deux compartiments à savoir un compartiment superficiel, l'épiderme, et un compartiment profond, le derme.

L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.

Le derme fournit à l'épiderme un support solide. C'est également son élément nourricier. Il est principalement constitué de fibroblastes et d'une matrice extracellulaire composée elle-même de différentes protéines extracellulaires parmi lesquelles figure notamment les fibres de collagène, l'élastine et différentes glycoprotéines. L'ensemble de ces composants extracellulaires est synthétisé par le fibroblaste. On trouve également dans le derme des leucocytes, des mastocytes ou encore des macrophages tissulaires. Enfin le derme contient des vaisseaux sanguins et des fibres nerveuses.

Le fibroblaste, de par son activité dans la synthèse des protéines extracellulaires matricielles (protéoglycannes, fibres de collagène et autres glycoprotéines de structure) est l'acteur principal de l'élaboration structurelle du derme.

Les fibres de collagène assurent la solidité du derme. Elles sont très résistantes mais sensibles à certaines enzymes appelées généralement collagénases. Dans le derme, les fibres de collagène sont constituées de fibrilles scellées les unes aux autres, formant ainsi plus de dix types de structures différentes. La structure du derme est en grande partie due à l'enchevêtrement des fibres de collagène tassées. Les fibres de collagène participent à la tonicité de la peau.
Les fibres de collagènes sont régulièrement renouvelées mais ce renouvellement diminue avec l'âge ce qui entraîne notamment un amincissement du derme. Il est également admis que des facteurs extrinsèques comme les rayons ultraviolets, le tabac ou certains traitements (Acide rétinoïque et dérivés, glucocorticoïdes, vitamine D et dérivés par exemple) ont également un effet sur la peau et sur son taux de collagène.

Au niveau de la composante dermique de la peau, la glycation intervient principalement dans le derme, sur les fibres de collagène, selon le processus décrit plus haut. La glycation du collagène augmente de façon régulière avec l'âge entraînant une augmentation régulière de la teneur de la peau en produits de glycation.

Sans vouloir introduire une quelconque théorie du vieillissement de la peau, il faut noter que d'autres modifications du collagène qui pourraient également être une conséquence de la glycation, comme une diminution de la dénaturation par la chaleur, une augmentation de la résistance à la digestion enzymatique et une augmentation des pontages intermoléculaires, ont pu être mises en évidence au cours du vieillissement de la peau ( Tanaka S. et col., 1988, J. Mol. Biol., 203, 495-505 ; Takahashi M. et col., 1995, Analytical Biochemistry, 232, 158-162 ). De plus, des modifications dues à la glycation de certains constituants de la membrane basale comme le collagène IV, la laminine et la fibronectine ont pu être mises en évidence (Tarsio JF. et col., 1985, Diabetes, 34, 477-484 ; Tarsio JF. et col, 1988, Diabetes, 37, 532-539 ; Sternberg M. et col., 1995, C. R. Soc. Biol., 189, 967-985 ).
Ainsi on comprend qu'au cours du vieillissement de la peau les propriétés physico-chimiques du collagène se modifient et ce dernier devient plus difficilement soluble et plus difficilement dégradable.
Ainsi une des composantes de la peau âgée apparaît bien être le collagène glyqué.

On sait très bien que la peau résulte d'une étroite association entre au moins deux compartiments qui la constituent à savoir l'épiderme et le derme. Les interactions entre le derme et l'épiderme sont telles qu'il est raisonnable de penser qu'une modification de l'un peut avoir des conséquences sur l'autre. On peut suspecter que le vieillissement du derme en particulier avec ses phénomènes de glycation ne peut qu'avoir des conséquences sur l'épiderme qui lui est associé: Ainsi au cours du vieillissement cutané, la glycation du collagène doit entraîner des modifications de l'épiderme qui participent nécessairement au vieillissement de l'épiderme.

Ainsi, si la glycation des protéines du derme, particulièrement du collagène entraîne autant de conséquences dommageables au niveau de la peau, des conséquences similaires sont a attendre de la glycation des protéines au niveau des annexes de la peau comme par exemple les ongles et/ou les cheveux et en fait au niveau de tout système protéique.

On comprend donc l'importance qui existe à disposer de produits qui diminuent voire inhibent le phénomène de glycation des protéines.

A cet égard la demanderesse a de manière surprenante et inattendue découvert que les hydroxystilbènes en général et le 3,3',5,5'-tétrahydroxystilbène ou de ses dérivés en particulier, présentent la propriété de diminuer voire inhiber le phénomène de glycation des protéines.

Le 3,3',5,5'-tétrahydroxystilbène de l'invention répond à la formule générale I: 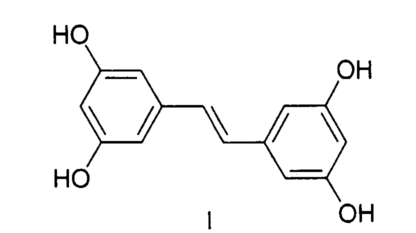 Ce composé peut être sous une forme Cis ou Trans.

Les hydroxystilbènes sont des composés que l'on retrouve à l'état naturel dans les végétaux de la classe des spermatophytes et particulièrement dans la vigne. On trouve de tels composés comme par exemple le resvératrol dans le raisin et dans le vin.

Dans l'art antérieur les hydroxystilbènes sont utilisés entre autres comme agents dépigmentants (JP87-192040), comme agents vasodilatateurs (EP 96-830517), comme agents antithrombiques (JP 05016413), dans le traitement de diverses affections cardio-vasculaires (CA 2187990), comme agents inhibiteurs de mutagenèse et de carcinogenèse (JP 06024967), ou encore décrits comme antioxydants.
A cet égard on peut consulter la revue de Soleas et collaborateurs (Clinical Biochemistry, vol. 30, N°2, pp. 91-113, 1997) qui résume parfaitement l'état des connaissances concernant les hydroxystilbènes.

Mais, la capacité du 3,3',5,5'-tétrahydroxystilbène ou de ses dérivés à diminuer voire inhiber le phénomène de glycation n'a jamais été décrite à ce jour.

L'invention a donc pour objet l'utilisation dans une composition ou pour la préparation d'une composition, d'une quantité efficace du 3,3',5,5'-tétrahydroxystilbène ou de ses dérivés, cet hydroxystilbène ou la composition étant destinés à diminuer voire inhiber la glycation des protéines, particulièrement la glycation des protéines de la peau et/ou de ses annexes.

Par "3,3',5,5'-tétrahydroxystilbène ou de ses dérivés", il faut entendre selon l'invention le 3,3',5,5'-tétrahydroxystilbène lui-même ou l'un de ses dérivés O-alkylés ou O-acylés.
Par dérivé O-alkylé, on entend selon l'invention que au moins l'une des fonctions phénol du 3,3',5,5'-tétrahydroxystilbène est substituée par un radical alkyle. Par radical alkyle on entend selon l'invention les radicaux acycliques provenant de l'enlèvement d'un atome d'hydrogène dans la molécule d'un hydrocarbure, linéaires ou ramifiés, éventuellement substitués, ayant de 1 à 10 atomes de carbone, préférentiellement ceux ayant de 1 à 4 atomes de carbone, en particulier les radicaux méthyle, éthyle, propyle, isopropyle, butyle, isobutyle ou tertiobutyle et plus particulièrement le radical méthyle. Par éventuellement substitué on entend selon l'invention une radical alkyle éventuellement substitué par un radical amino ou hydroxyle.

Par dérivé O-acylés on entend selon l'invention que au moins l'une des fonctions phénol du 3,3',5,5'-tétrahydroxystilbène est substituée par un radical acyle ayant de 1 à 10 atomes de carbone préférentiellement de 1 à 4, en particulier les radicaux acétyle, propanoyle, isopropanoyle, butanoyle, isobutanoyle, ter-butanoyle. Ce radical acyle peut éventuellement être substitué par un radical amino ou hydroxyle.

Très particulièrement, l'invention a pour objet l'utilisation dans une composition ou pour la préparation d'une composition, d'une quantité efficace du 3,3',5,5'-tétrahydroxystilbène ou de ses dérivés, cet hydroxystilbène ou la composition étant destinés à diminuer voire inhiber la glycation des protéines du derme, comme par exemple le collagène, et/ou des ongles et/ou des cheveux, comme par exemple les kératines.

L'invention a en outre pour objet l'utilisation dans une composition ou pour la préparation d'une composition, d'une quantité efficace du 3,3',5,5'-tétrahydroxystilbène ou de ses dérivés, cet hydroxystilbène ou la composition étant destinés à traiter, de manière préventive et/ou curative, les signes du vieillissement de la peau ou de ses annexes liés à la glycation.

Par voie de synthèse chimique, le 3,3',5,5'-tétrahydroxystilbène, composé connu, peut être préparé selon la méthode décrites dans Journal of Medicinal Chemistry, 1993, Vol. 36, n°20, page 2950.

Selon l'invention le 3,3',5,5'-tétrahydroxystilbène ou de ses dérivés peut être utilisé seul ou en mélanges de toute nature soit avec ces dérivés hydroxyalkylés soit avec d'autres hydroxystilbènes et/ou leur dérivés hydroxyalkylés.

Particulièrement le 3,3',5,5'-tétrahydroxystilbène ou l'un de ses dérivés ou la composition le contenant sont utilisés selon l'invention en application topique sur la peau et/ou les ongles et/ou les cheveux.

La quantité de 3,3',5,5'-tétrahydroxystilbène ou de ses dérivés utilisable selon l'invention dépend bien évidemment de l'effet recherché et doit être en une quantité efficace pour diminuer voire inhiber la glycation.

A titre d'exemple, la quantité de 3,3',5,5'-tétrahydroxystilbène ou de ses dérivés utilisable selon l'invention peut aller par exemple de 0,001% à 10% et de préférence de 0,005% à 5% du poids total de la composition.

En outre, la composition de l'invention est utilisée pendant un temps suffisant pour obtenir les effets attendus selon l'invention. Pour donner un ordre de grandeur, cette durée peut être au minimum de 3 semaines, mais peut être aussi de plus de 4 semaines, voire de plus de 8 semaines.

La composition est de préférence destinée à un usage cosmétique ou dermatologique, avantageusement cosmétique.

La composition de l'invention destinée à une application topique contient un milieu physiologiquement acceptable, c'est-à-dire compatible avec la peau y compris le cuir chevelu, ses annexes, les muqueuses et/ou les yeux et peut constituer notamment une composition cosmétique ou dermatologique.

Cette composition peut se présenter sous toutes les formes galéniques normalement utilisées dans les domaines cosmétique et dermatologique, et elle peut être notamment sous forme d'une solution aqueuse éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles.

La composition de l'invention peut constituer par exemple une lotion, un gel, une crème ou un lait, et par exemple une lotion ou un lait de démaquillage ou de nettoyage, un shampooing ou un gel douche.

L'invention a également pour objet un procédé de traitement cosmétique pour traiter les signes du vieillissement liés à la glycation des protéines, particulièrement de la peau et/ou des ongles et/ou des cheveux, caractérisé par le fait que l'on applique sur la peau et/ou les ongles et/ou les cheveux une composition cosmétique comprenant une quantité efficace de 3,3',5,5'tétrahydroxystilbène ou de ses dérivés, cet hydroxystilbène ou la composition étant destinés à inhiber la glycation.

D'autres caractéristiques et avantages de l'invention ressortiront mieux des exemples qui suivent, donnés à titre illustratif et non limitatif. Dans ce qui suit ou ce qui précède, les proportions sont données en pourcentage pondéral, sauf indications contraires.

Exemple 1 : Etude de l'effet du 3,3',5,5'-tétrahydroxystilbène sur la glycation, en comparaison de l'effet produit par le resvératrol.

Une solution de sérum albumine bovine à 1 mg/ml en solution dans du tampon phosphate salin (PBS) est incubée à 37°C pendant 14 jours en présence ou en absence de D-ribose à la concentration de 10mM, de 3,3',5,5'-tétrahydroxystilbène ou de resvératrol, chacun aux concentrations de 1µM et de 10µM.
La glycation est évaluée en mesurant la fluorescence des AGEs à λem. = 440 nm émise par chaque échantillon après excitation à λex. = 370 nm.
L'inhibition de la glycation est visualisée par la diminution de la fluorescence comparée à l'échantillon traité avec le sucre seul.

Les résultats sont :

| | A : 1µM | B : 1µM | A : 10µM | B : 10µM |
|---|---|---|---|---|
| % inhibition | 7,1% | 7,1% | 14,3% | 7,1% |
| A : 3,3',5,5'-tétrahydroxystilbène | | | | |
| B : resvératrol | | | | |

Le 3,3',5,5'-tétrahydroxystilbène présente un effet anti-glycation intéressant dès la concentration de 1 µM.
Cet effet est au moins égal à celui du resvératrol à faible concentration et apparaît 2 fois supérieur à la concentration de 10µM.

Exemple 2 : Exemples de formulations illustrant l'invention et particulièrement les compositions selon l'invention. Ces compositions ont été obtenues par simple mélange des différents composants.

| Compositon 1 : Lotion démaquillante pour le visage | |
|---|---|
| 3,3',5,5'-tétrahydroxystilbène | 0,05 % |
| Antioxydant | 0,05 % |
| Isopropanol | 40,00 % |
| Conservateur | 0,30 % |
| Eau qsp | 100,00 % |

| Composition 2 : Shampooing | |
|---|---|
| 3,3',5,5'-tétrahydroxystilbène | 1,00 % |
| Hydroxypropylcellulose (Klucel H® vendu par la société | Hercules) 1,00 % |
| Lauryl sulfate de sodium | 12,00 % |
| Parfum | 0,50 % |
| Conservateur | 0,30 % |
| Eau qsp | 100,00 % |

| Composition 3 : Crème de soin du visage (émulsion huile dans eau) | |
|---|---|
| 3,3',5,5'-tétrahydroxystilbène | 0.005 % |
| Stéarate de glycérol | 2,000 % |
| Polysorbate 60 (Tween 60® vendu par la société ICI) | 1,000 % |
| Acide stéarique | 1,400 % |
| Triéthanolamine | 0,700 % |
| Carbomer | 0,400 % |
| Fraction liquide du beurre de karité | 12,000 % |
| Perhydrosqualène | 12,000 % |
| Antioxydant | 0,050 % |
| Parfum | 0,500 % |
| Conservateur | 0,300 % |
| Eau qsp | 100,00 % |

| Composition 4 : Gel pour la peau | |
|---|---|
| 3,3',5,5'-tétrahydroxystilbène | 2,00 % |
| Acide tout trans rétinoïque | 0,05 % |
| Hydroxypropylcellulose (Klucel H® vendu par la société Hercules) | 1,00 % |
| Antioxydant | 0,05 % |
| Isopropanol | 40,00 % |
| Conservateur | 0,30 % |
| Eau qsp | 100,00 % |

| Composition 5 : Gel pour le soin du visage | |
|---|---|
| 3,3',5,5'-tétrahydroxystilbène | 0,01 % |
| Hydroxypropylcellulose (Klucel H® vendu par la société | Hercules) 1,00 % |
| Antioxydant | 0,05 % |
| Isopropanol | 40,00 % |
| Conservateur | 0,30 % |
| Eau qsp | 100,00 % |

| Composition 6 : Gel | |
|---|---|
| 3,3',5,5'-tétrahydroxystilbène | 0,10 % |
| Hydroxypropylcellulose (Klucel H® vendu par la société | Hercules) 1,00 % |
| Antioxydant | 0,05 % |
| Chlorhydrate de lidocaïne | 2,00 % |
| Isopropanol - | 40,00 % |
| Conservateur | 0,30 % |
| Eau qsp | 100,00 % |

| Composition 7 : Crème de soin (émulsion huile-dans-eau) | |
|---|---|
| 3,3',5,5'-tétrahydroxystilbène | 5,00 % |
| Stéarate de glycérol | 2,00 % |
| Polysorbate 60 (Tween 60® vendu par la société ICI) | 1,00 % |
| Acide stéarique | 1,40 % |
| Acide glycyrrhétinique | 2,00 % |
| Triéthanolamine | 0,70 % |
| Carbomer | 0,40 % |
| Fraction liquide du beurre de karité | 12,00 % |
| Huile de tournesol | 10,00 % |
| Antioxydant | 0,05 % |
| Parfum | 0,50 % |
| Conservateur | 0,30 % |
| Eau qsp | 100,00 % |

## Revendications

1. Utilisation dans une composition ou pour la préparation d'une composition d'une quantité efficace de 3,3',5,5'-tétrahydroxystilbène ou de l'un de ses dérivés, cet hydroxystilbène ou la composition étant destinés à diminuer la glycation.

2. Utilisation selon la revendication précédente, caractérisée par le fait que la glycation est la glycation des protéines.

3. Utilisation selon l'une quelconque des revendications précédentes, caractérisée par le fait que les protéines sont les protéines de la peau et/ou des ongles et/ou des cheveux.

4. Utilisation selon la revendication précédente, caractérisée par le fait que les protéines de la peau sont les protéines du derme.

5. Utilisation selon la revendication précédente, pour diminuer la glycation du collagène.

6. Utilisation selon la revendication 3, caractérisée par le fait que les protéines des ongles et/ou des cheveux sont les kératines.

7. Utilisation selon l'une quelconque des revendications précédentes pour traiter, de manière préventive et/ou curative, les signes du vieillissement de la peau et/ou de ses annexes liés à la glycation.

8. Utilisation selon l'une quelconque des revendications précédentes, caractérisée par le fait que le 3,3',5,5'-tétrahydroxystilbène ou l'un de ses dérivés, est présent en une quantité allant de 0,001% à 10% du poids total de la composition

9. Utilisation selon la revendication précédente, caractérisée par le fait que le 3,3',5,5'-tétrahydroxystilbène ou l'un de ses dérivés, est présent en une quantité allant de 0,005.% à 5 % du poids total de la composition.

10. Procédé de traitement cosmétique pour traiter les signes du vieillissement liés à la glycation des protéines de la peau et/ou des ongles et/ou des cheveux, caractérisé par le fait que l'on applique sur la peau et/ou les ongles et/ou les cheveux une composition cosmétique comprenant une quantité efficace de 3,3',5,5'-tétrahydroxystilbène ou de l'un de ses dérivés, cet hydroxystilbène ou la composition étant destinés à inhiber la glycation.
